# EUROPEAN PATENT SPECIFICATION

(11) **EP 1 225 449 B1**
(45) Date of publication and mention of the grant of the patent: **22.10.2008**
(21) Application number: 01300476.7
(22) Date of filing: 19.01.2001
(51) Int. Cl.: G01N 33/49, G01N 33/72, G01N 33/52, G01N 27/26

(54) **Non-enzymatic disposable electrode strip comprising a surfactant for detecting uric acid or hemoglobin; method for producing the same and its use**
Nicht-enzymatischer, Detergenzien enthaltender Einweg-Elektrodestreifen für den Nachweis von Harnsäure oder Hämoglobin,sowie Verfahren für die Produktion und Anwendung desgleichen
Electrode non-enzymatique et disposable contenant un tensioactif pour la détection d'acide urique ou d'hémoglobine; méthode pour le produire et son utilisation

(43) Date of publication of application: 24.07.2002
(60) Divisional application: 08005522.1
(73) Proprietor: Apex Biotechnology Corporation, Hsinchu Science Park Hsinchu (TW)
(72) Inventor: Lin, Yueh-Hui, Hsinchu (TW); Shen, Thomas Y.S., Hsinchu (TW)
(74) Representative: Woods, Geoffrey Corlett

(56) References cited:
- EP-A- 1 025 758
- US-A- 4 301 412
- US-A- 4 713 165
- US-A- 4 876 205
- US-A- 5 556 533
- US-A- 5 779 867
- US-A- 6 054 039
- DATABASE WPI Section Ch, Week 200035 Derwent Publications Ltd., London, GB; Class B04, AN 2000-409729 XP002175754 & TW 369 411 A (WU DING BIOTECHNOLOGY CO LTD), 11 September 1999 (1999-09-11) & US 6 258 223 B1 (APEX BIOTECHNOLOGY CORP.) 10 July 2001 (2001-07-10)
- DATABASE WPI Section Ch, Week 200132 Derwent Publications Ltd., London, GB; Class B04, AN 2001-307008 XP002175755 & TW 416 005 A (APEX BIOTECHNOLOGY CORP), 21 December 2000 (2000-12-21) & DE 100 18 959 A (APEX BIOTECHNOLOGY CORP) 28 June 2001 (2001-06-28)
- Productinformation from SIGMA about Triton X-100 dated 21.04.1999.

## Description

The present invention relates to a disposable electrode strip for detecting biomolecules, particularly hemoglobin.

The detection of biomolecules can be carried out by two types of electrodes: enzyme electrode and non-enzyme electrode. For clinical biochemical analysis, biomolecules are detected through a reduction or an enzymatic method. Enzyme electrodes cannot be mass-produced because of the high costs caused by strict preservation conditions, complicated manufacturing procedures and control conditions. Enzyme electrodes are not disposable and suitable for household use. On the contrary, non-enzyme electrodes can be made of disposable material and are easy to produce, store and use; therefore, they are advantageous bio-analyzers for detecting biomolecules such as uric acid and hemoglobin.

Up to now, there are no effective disposable electrode strip for the detection of the uric acid and hemoglobin. Ai-min Yu, Hai-li Zhang and Hong-yuan Chen (Analyst, 1997; 122:839~841) described the improved voltammeric behavior of uric acid and its trace determination at a polyglycine chemically modified electrode. The electrode is merely tested in the sample of uric acid in a 0.1 M phosphate buffer rather than the whole-blood sample. Such electrode has the disadvantages as follows: (1) the linear ranges are out of normal diagnostic ranges; (2) such electrode is not suitable for detecting blood sample; (3) the electrode manufacturing process is time-consuming and complicated and not suitable for mass production, and (4) the electrode costs high, and thus can not be used as a disposable strip.

Jyh-Myng Zen and Jen-Sen Tang (Anal. Chem. 1995; 67:1892∼1895) modified the glassy carbon electrode by Nafion/Ru₂₋ₓPbₓO₇₋ₓ (ruthenium oxide pyrochlore) and detected uric acid by Osteryoung square-wave voltammeter. The detection of the electrode is suitable in an acidic uric acid solution with pH 1. The electrode is not suitable for detecting blood sample. Moreover, such electrode has the disadvantages as follows: (1) the price of the electrode is expensive; (2) the detectable ranges do not meet the diagnostic detection ranges; (3) the electrode manufacturing process is complex and not suitable for mass production; and (4) the electrode can not be used as a disposable strip and thus is not suitable for household use.

As to the detection of the hemoglobin, the Cyanmet-hemoglobin method is commonly used in the art. Said method uses Drabkin's solution to solve hemoglobin, i.e. an oxidization of the Fe²⁺ of hemoglobin to Fe³⁺ with K₃Fe(CN)₆ to form met-hemoglobin (MHb). Further, said MHb combines with KCN to form a stabilized cyanmet-hemoglobin. The concentration of hemoglobin can be obtained by measuring absorption at. 540nm using optical colorimetry. Based on the oxidization of Fe²⁺ of hemoglobin with K₃Fe(CN)₆, the hemoglobin can be detected through a suitable electrode and a detectable oxidation potential. However, the above-mentioned method has a disadvantage of causing pollution of KCN having a high toxicity.

US - A- 4876205 describes an electrochemical assay for haemoglobin. US-A-5556533 describes a method for applying voltage to a hydrogen peroxide-type enzyme electrode.

Given the above, there is a need to develop a disposable, easy-to-use and inexpensive electrode strip in the detection of biomolecules, in particular hemoglobin.

### SUMMARY OF THE INVENTION

One object of the invention is to provide a hemoglobin disposable detecting electrode strip, comprising an electric insulating substrate, a conducting film, an electric insulating film and a reaction film.

Another object of the invention is to provide a method for producing a disposable hemoglobin detecting electrode strip

Another object of the invention is to provide a hemoglobin detecting equipment.

Another further object of the invention is to provide a method for detecting the concentration of hemoglobin in a liquid sample.

### BRIEF DESCRIPTION OF THE DRAWINGS

FIG.1a is a top view diagram of an electrode strip of the present invention.

FIG.1b is a front view diagram of an electrode strip of the present invention.

FIG.2a shows the step of coating the conducting film 2 on electric insulating substrate 1 to form at least an anode and a cathode.

FIG.2b shows the step of partially covering an electric insulating film on the conducting film. The uncovered conducting film contains an anode connector 6, a cathode connector 7, a working electrode 8 and a reference electrode 9.

FIG.2c shows the step of coating a reaction film 4 on a reaction region formed by coating working electrode and the reference electrode.

FIG.2d shows the step of coating a reaction film with a protection film 5.

FIG.3a shows the hemolysis caused by the reaction film of the hemoglobin detecting electrode strip.

FIG. 3b shows the concentrations of the uric acid and hemoglobin can be measured through the adjustment of current of the detection device.

FIG. 4 shows the concentration of hemoglobin in whole-blood respectively detected by the electrode strip of the present invention and Cyanmet-hemoglobin method.

FIG. 5 shows the hematocrit in whole-blood respectively detected by the electrode strip of the present invention and the centrifugation method.

### BRIEF DESCRIPTION OF THE ELEMENTS NUMERALS

- 1: Insulating substrate
- 2: Conducting film
- 3: Insulating film
- 4: Reaction film
- 5: Protection film
- 6: Anode connector
- 7: Cathode connector
- 8: Working electrode
- 9: Reference electrode

### DETAILED DESCRIPTION OF THE INVENTION

The present invention features a disposable detecting electrode strip modified by a water-soluble redox electron mediator and a surfactant. The electrode strip detects the concentration of biomolecules in a liquid sample by catching signals generated by a redox current of a redox electron mediator and the biomolecules such as uric acid and hemoglobin.

It is surprisingly found that the electrode modified by a higher concentration of neutral surfactant Triton X-100 has a high reaction velocity and accuracy in the detection of hemoglobin.

### Electrode Article

An object of the invention is to provide a disposable electrode article eg. in the form of a strip which is suitable for the non-enzymatic detection of hemoglobin, which article comprises:
(1) an electric insulating substrate;
(2) a conducting film coated on one side of said insulating substrate to form an isolated and disconnected an anode and a cathode;
(3) an electric insulating film coated on a part of said conducting film, wherein one end of an uncovered anode of the conducting film forms at least a reference electrode and the other end an anode connector, and one end of an uncovered cathode of the conducting film forms at least a working electrode and the other end a cathode connector, and
(4) a reaction film comprising a carrier, a conductive mediator and a neutral surfactant Triton X-100 with the concentration of more than 1%, and being coated on a region containing at least said working electrode and said reference electrode so as to connect said working electrode and said reference electrode individually.

According to the invention, the electric insulating substrate has a flat surface and an insulation property. Said substrate is thermal-resistant more than 40°C, preferably 40-80°C, which can increase the conductivity through thermal processing. Any materials with the above-mentioned properties are suitable for the electric insulating substrate of the invention. Preferably, such materials are selected from the group consisting of polyvinyl chloride (PVC), fiber glass (FR-4), polyester sulphone, bakelite plate, polyethylene terephthalate (PET) plate, polycarbonate (PC) plate. glass plate and ceramics plate (CEM-1).

According to the invention, the conducting film is coated on one side of said insulting substrate to form an isolated and disconnected cathode and an isolated and disconnected anode. Preferably, the coating is achieved by screen printing with printing ink or sticking with a metal film. More preferably, the metal film is selected from the group consisting of gold, silver, platinum and palladium. The printing ink is selected from the group consisting of carbon ink, gold ink, silver ink, the mixture of carbon and silver ink, volatile graphite, copper ink, or the mixture of the above (for example, printing silver ink first and then printing carbon ink). Said cathode is partially covered by said electric insulating film and the uncovered ends of the cathode form a reference electrode and a cathode connector, respectively. The anode is also partially covered by said electric insulating film and the uncovered ends of the anode form a working electrode and anode connector respectively. The reference electrode of the cathode is covered by the reaction film and cooperates together with the working electrode of the anode to detect the induced electric effect. The cathode and anode connectors are used to connect with the amperometric sensor.

According to the invention, the electric insulating film is coated on one surface of said electric insulating substrate but does not insulate the above-mentioned cathode connector, anode connector, working electrode and reference electrode. The region uncovered by the electric insulating film including the working electrode and the reference electrode forms a reaction region, which is then coated by the reaction film for testing samples. Typically the thickness of the electric insulating films is 0.1mm to 2mm, preferably more than 0.6 mm.

According to the invention, the reaction film is coated on the reaction region by any suitable method, preferably by pipetting or screen printing. The reaction film consists of a water-soluble redox electron mediator, a carrier and a surfactant. The reaction film contacts with a sample and reacts electrochemically. Further, the reaction film can be covered with a protection film.

The conductive mediator is a material for donating or accepting electrons, that can be used as the mediator for transferring electron between the tested analyte and electrode in the redox reaction. The conductive mediator will change from oxidation state to reduction state after reacting with tested sample. The conductive mediator can then reverse to oxidation state by applying a forced voltage. The changes of, such as potential, resistance, or current caused by electrochemical reaction, can be transferred through the conducting film, i.e. from the working electrode and the reference electrode connected with the reaction film to the cathode connector and the anode connector. Typically therefore a conductive mediator suitable for 100mV to 500mV of redox potential difference can be used in the invention.

Preferably, said conductive mediator is selected from the group consisting of potassium ferricyanide, ferricinium, tetrathisfulvalene, methylviologen and ferricyanide. More preferably, said conductive mediator is potassium ferricyanide. The amount of said conductive mediator preferably ranges from 0.3 to 5% by weight of the reaction film.

According to the invention, the carrier absorbs a sample because the sample is hydrophilic and hard to attach to a hydrophobic conducting film. The carrier can enhance the absorption of sample, and enhance the signals to be transferred to the conducting film. Said reaction film contacts with a sample and reacts electrochemically with said sample. Therefore, the carrier can be distributed all over the reaction film area and the signals be thoroughly transferred. Preferably, the carrier is selected from the group consisting of microcrystalline cellulose, carboxymethyl cellulose or methyl cellulose. More preferably, the celluloses have a size of below 100 µm. Preferably, the amount of the celluloses ranges from about 0.01% to about 0.1 % by weight of the reaction film.

According to the invention, the carrier optionally comprises a polymer. The polymer is to facilitate the reaction film having a certain viscosity. The reaction film can therefore be distributed thoroughly. Preferably, the polymer is selected from the group consisting of polyvinyl alcohol (PVA), polyvinyl pyrrolidone (PVP), polyethylene glycol (PEG), gelatin and the mixture thereof. More preferably, the amount of the polymer ranges from 0 to 15% by weight of the reaction film.

According to the invention, for the detection of hemoglobin, the surfactant used in said reaction film is a neutral surfactant with the concentration more than 1% by weight eg. 1% to 6% by weight.

The surfactant is triton. Most preferably, the concentration of said neutral surfactant ranges from 1% to 5% by weight.

Further information about surfactants may be found for example, in "McCutcheon's Detergents and Emulsifiers Annual", MC Publishing Corp. Ringwood, New Jersey, 1979, and Sisely and Wood, "Encyclopaedia of Surface Active Agents," Chemical Publishing Co., Inc. New York, 1980.

According to the invention, the non-enzymatic disposable hemoglobin detecting electrode strip can be used in the detection of hematocrit. Persons skilled in the art know that the ratio value of hematocrit/ hemoglobin is between 2.9 and 3.1. Therefore, the hematocrit can be calculated by the concentration of hemoglobin detected by the hemoglobin detecting electrode strip of the invention.

### Preparation of Electrode Strip

Another object of the invention is to provide a method for producing disposable non-enzymatic or hemoglobin detecting electrode strip which comprises:
(a) coating a conducting film on one side of an electric insulating substrate and forming isolated and disconnected an anode and a cathode;
(b) coating an electric insulating film on a part of the conducting film, wherein one end of an uncovered anode of the conducting film is at least a reference electrode and the other end an anode connector, and one end of an uncovered cathode of the conducting film is at least a working electrode and the other end a cathode connector; and
(c) screen printing or pipetting a reaction film on a region containing at least the working electrode and the reference electrode so as to connect the working electrode and the reference electrode individually.

According to the invention, first, a conducting film is coated on one side of a flat substrate to form at least an anode and a cathode that are separately isolated from each other. After coating, the conducting film is dried at 35°C to 80°C.

According to the invention, secondly, an electric insulating film with a thickness of 0.6 mm or above is partially printed onto the conducting film. The uncovered parts of the conducting film form a cathode connector, an anode connector, a working electrode and a reference electrode. An area formed by the working electrode and the reference electrode in a circle or any other suitable shape is an area of reaction film.

According to the invention, thirdly, a reaction film is pipetting or screen printing on a reaction region so that the individual working electrode and the reference electrode can be connected. Said region can be a circle or any other suitable shape. Several conventional screen printing technologies can be used in the invention. Moreover, a new screen printing technology disclosed by one of the inventors of the present invention and contained in R.O.C. Patent Application Number 85,109,554 could also be applied in the production method of the present invention.

According to the invention, fourthly, the reaction film can be further dried at 40 to 80° C. A protection film is optionally coated on and around the area of the reaction film. The disposable non-enzymatic electrode strip is therefore produced.

### Detection Equipment

Another further object is to provide a hemoglobin detecting equipment comprises disposable non-enzymatic hemoglobin detecting electrode strip and a amperometric sensor directly analyzing hemoglobin in a liquid sample,
wherein the electrode strip comprises:
(1) an electric insulating substrate:
(2) a conducting film coated on one side of said insulting substrate to form an isolated and disconnected anode and an isolated and disconnected cathode;
(3) an electric insulating film coated on a part of said conducting film, wherein one end of an uncovered anode of the conducting film forms at least a reference electrode and the other end of an anode connector, and one end of an uncovered cathode of the conducting film forms at least a working electrode and the other end a cathode connector; and
(4) a reaction film comprising a carrier, a conductive mediator and a neutral surfactant Triton X-100 with the concentration of more than 1 %, and being coated on a region containing at least said working electrode and said reference electrode so as to connect said working electrode and said reference electrode individually wherein said reaction film does not contain an enzyme; and wherein the amperometric sensor is connected to the anode connector and the cathode connector of the hemoglobin detecting electrode strip and comprises a voltage output equipment, a signal receiver and a display equipment; in which the voltage output equipment provides a voltage of below 400 mV to the reaction film of the hemoglobin detecting electrode strip so as to oxidize the conductive mediator from reduction state to oxidation state after being reacted with hemoglobin of the sample; the signal receiver receives a current, voltage or resistance change generated during a redox reaction and transmits the change to the display equipment to display the concentration of hemoglobin in the sample.

According to the invention, the detecting conditions at a low operation voltage of below 400 mV and at a pH value from 5.0 to 10.0 avoid interference caused by other oxidizing components.

### Detection Method

Further object of the invention is to provide a method for detecting a concentration of hemoglobin in liquid, which comprises dropping the liquid on a non-enzymatic disposable hemoglobin detecting electrode strip of the invention, and controlling an amperometric sensor to be operated at a low operation voltage of below 400 mV, and a pH value in the range from 5.0 to 8.0.

According to the invention, the detecting method of non-enzymatic electrode strip can proceed easily by using electrochemical detecting equipment. The reaction current caused by the redox reaction of hemoglobin can be detected in a hemoglobin detecting equipment by dropping a whole-blood sample on the reaction area of the non-enzymatic disposable hemoglobin detecting electrode strip of the present invention. Such an electrochemical reaction technology is commonly applied in electrochemical blood sugar monitor for detecting blood sugar. The method of directly detecting hemoglobin in whole-blood is novel and first disclosed in the present invention by using a redox electron mediator to transfer signals of the redox reaction of hemoglobin and by controlling the reaction at a pH value from 5.0 to 8.0 as well as a low operation voltage of below 400 mV to avoid the interference result from glucose and ascorbic acid in blood.

The present invention can be used in the detection of biomolecules such as hemoglobin in any liquid samples. The sample which may be tested in accordance with the invention may be any body fluid, preferably blood or a component of blood, more preferably whole blood. When taking whole-blood as a sample for detecting hemoglobin, there will be interference caused by other components, especially ascorbic acid in the blood. The disposable detecting electrode strip and detecting equipment of the present invention can directly detect the hemoglobin in whole-blood for household use.

One important advantage of the present invention is to eliminate the use of any bio-active substances such as enzymes. So, the present invention simplifies the production process, reduces the production cost, increases the storage term, and releases the storage restriction of the detecting electrode strip.

### DETAILED DESCRIPTIONS OF PREFERRED EMBODIMENT OF THE INVENTION

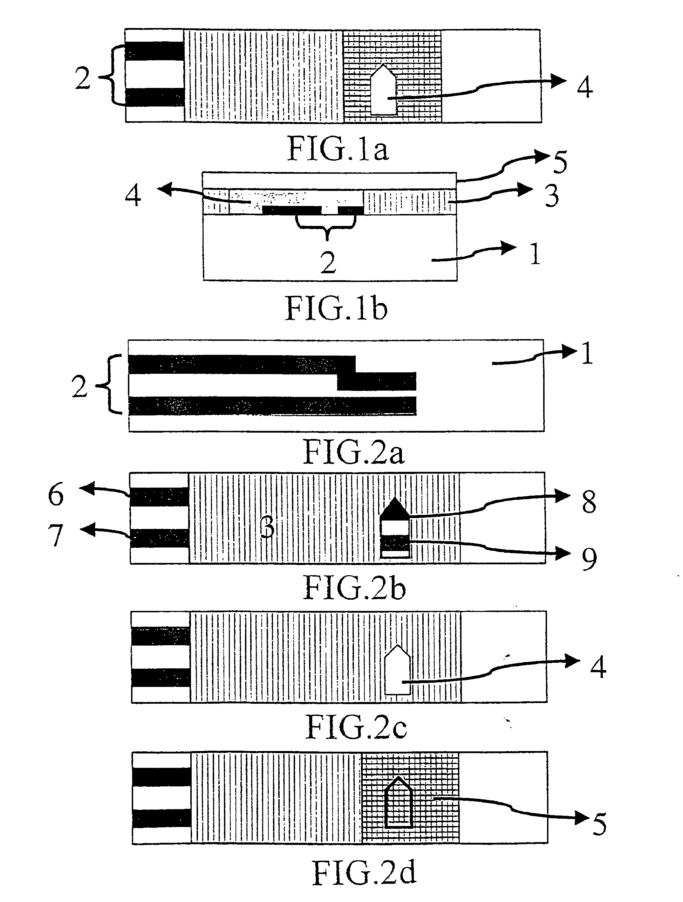

Figs. 1a and 1b are respectively a top view diagram and a front view diagram of an electrode strip of the present invention. The electrode strip structure comprises an electric insulating substrate **1**, a conducting film **2** coated on the insulating substrate, an electric insulating film **3** coated on a part of the conducting film **2**, and a reaction film **4** for reacting with a sample.

The electric insulating substrate **1** of the present invention adheres to the conducting film **2**. The cathode of the conducting film **2** is partially covered by the electric insulating film 3 and two uncovered ends of the cathode are a working electrode **8** and a cathode connector **6** respectively. The working electrode **8** of the cathode is covered by the reaction film **4** and is used to detect an induced electric effect of samples during the electrochemical reaction of uric acid. The cathode connector **6** is used to connect with a amperometric sensor. The anode is also partially covered by the electric insulating film **3** and two uncovered ends of the anode are a reference electrode **9** and an anode connector **7** respectively. The reference electrode **9** of the anode is covered by the reaction film **4** and cooperates together with the working electrode of the cathode to detect the induced electric effect.

The preparation of the electrode strip is illustrated in Figures 2a, 2b, 2c and 2d. As shown in Figure 2a, a conducting film **2** is first coated such as by screen printing on one side of a flat substrate **1** to form at least an anode and a cathode which are separately isolated from each other.

As shown in Figure 2b, the uncovered parts of the conducting film form a cathode connector **6**, an anode connector **7**, a working electrode **8** and a reference electrode **9**. An area formed by the working electrode **8** and the reference electrode **9** in a circle.

Further, as shown in Figures 2c and 2d, a conductive mediator are screen printing on the circle area of the reaction film **4** and a protection film **5** is optionally coated on and around the circle area of the reaction film **4**.

The following examples are exemplified to describe the present invention in detail but not to confine the present invention.

### Example 1 Detection of Biochemical Materials

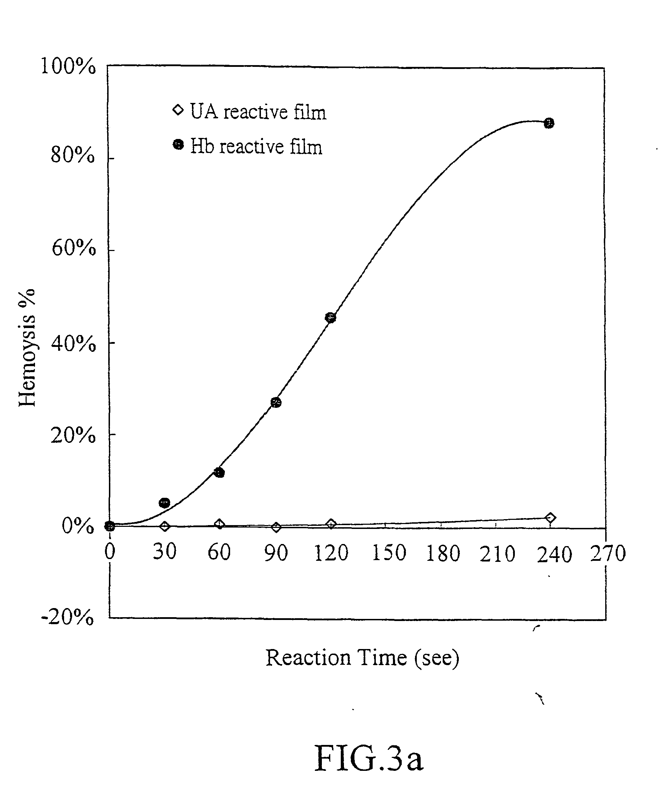

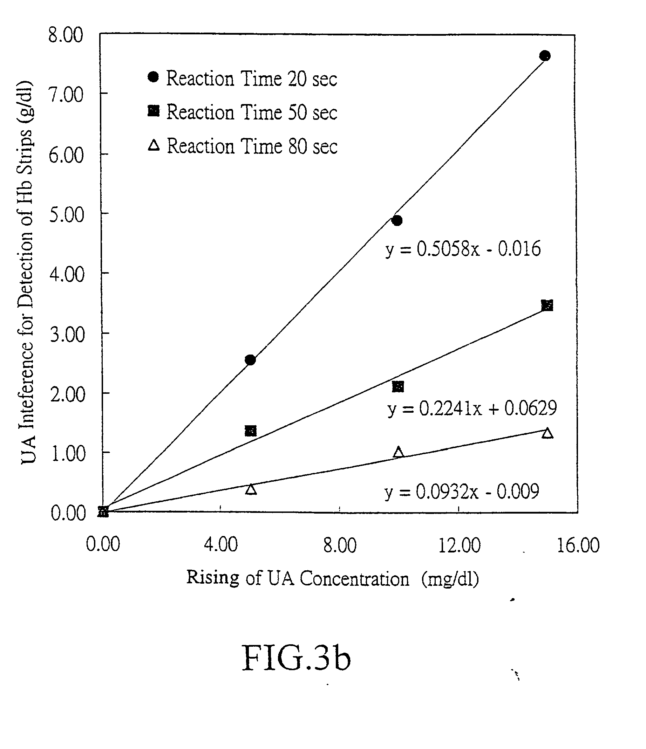

The detection of hemoglobin was controlled at a pH value from 7.0 to 10.0 as well as a low operation voltage of below 400 mV to avoid the interference result from glucose and ascorbic acid in blood. It found that the vitamin C within the normal concentration would not affect the measurement of the hemoglobin. Thus, the biochemical materials can be accuracy determined. Figure 3a shows the hemolysis caused by the reaction film of the hemoglobin detecting electrode strip. Figure 3b shows that the signal of the hemoglobin reaction can be amplified through the adjustment of current to reduce the interference of the uric acid.

### Example 2 (not of the invention) The Detection of Uric Acid

On one flat side of polyvinyl chlorine (PVC) substrate, carbon ink was screen printed to form a conducting film **2** comprising a set of isolated and disconnected anode and cathode and then dried at 40 to 80° C. An electric insulating film **3** with a thickness of about 0.6 mm was subsequently screen printed on the conducting film **2** partially. The uncovered part of the conducting film formed a cathode connector 6, a anode connector **7**, a working electrode **8** and a reference electrode **9**. The circle area formed by working electrode **8** and reference electrode **9** was an area of reaction film **4**.

Slurry materials comprising the following components and proportions were then pointed with pipet on the circle area of reaction film **4**.

| | |
|---|---|
| Methyl cellulose | 0.01 % |
| CTAB (N-acetyl-N,N,N-trimethyl-ammonium bromide) | 0.01 % |
| H₂O | 99.68 % |
| Potassium ferricyanide | 0.3 % |

After pointing said material on the reaction film **4**, it was dried at 35 to 80° C. A protection film **5** was coated on and around the circle area of reaction film **4**. The disposable non-enzymatic uric acid detecting electrode strip was produced through the production steps of the above.

The uric acid in blood was detected for 20 seconds by the above-obtained disposable non-enzymatic uric acid detecting electrode strip of the present invention by taking whole-blood as a sample. The test results were the same as the uric acid concentration detected by any conventional method.

### Example 3 (not of the invention) The Detection of Uric Acid

The steps of example 2 were repeated, except that the slurry materials were screen printed on the circle area of reaction film and the components and proportions of slurry materials were changed as follows:

| | |
|---|---|
| Methyl cellulose | 0.05 % |
| PEG, polyethylene glycol | 15.00 % |
| CTAB (N-acetyl-N,N,N-trimethyl-ammonium bromide) | 0.03 % |
| H₂O | 83.092 % |
| Potassium ferricyanide | 1.00 % |

The uric acid in blood was detected for 30 seconds by the above obtained disposable non-enzymatic uric acid detecting electrode strip of the present invention by taking whole-blood as a sample. The test results were the same as the uric acid concentration detected by uricase method in combination with EPAC bio-analyzer.

### Example 4 The Detection of Hemoglobin and Hemocrit

The steps of example 2 were repeated, except that the components and proportions of slurry materials were changed as follows:

| | |
|---|---|
| Methyl cellulose | 0.02 % |
| Triton X 100 | 4.00 % |
| H₂O | 92.98 % |
| Potassium ferricyanide | 3.00 % |

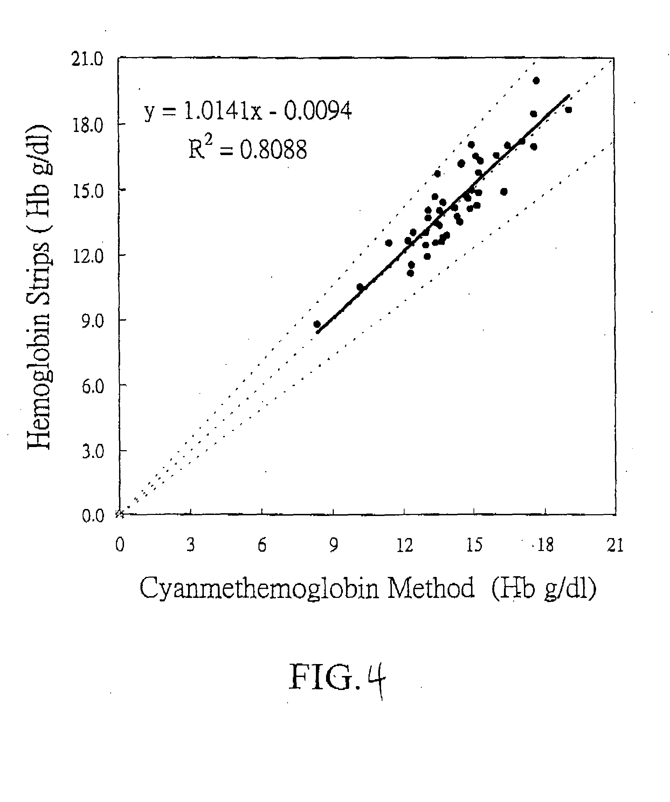

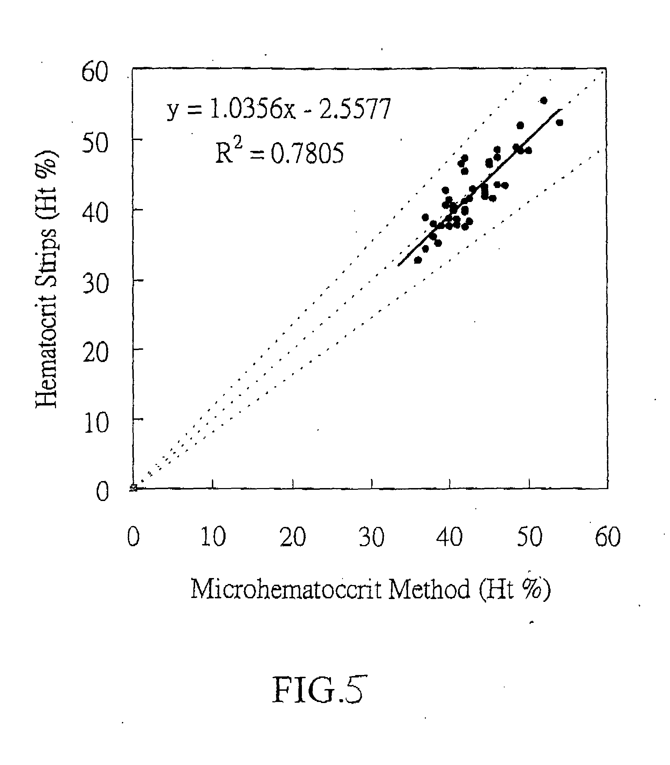

The hemoglobin in blood was detected for 30 seconds by the above obtained disposable non-enzymatic hemoglobin detecting electrode strip of the present invention by taking whole-blood as a sample. As shown in Figure 4, the test results were the same as the hemoglobin concentration detected by the cyanmet-hemoglobin method. In addition, the results of the hemocrit detected by the strip of the invention are same as those detected by centrifugation (see Figure 5).

The results show that the disposable non-enzymatic hemoglobin detecting electrode strip of the present invention can precisely detect the hemoglobin concentration and hemocrit in blood by taking whole-blood as a sample and does not require any pretreatment.

### Example 5 The Detection of Hemoglobin and Hemocrit

The steps of example 3 were repeated, except that the components and proportions of slurry materials were changed as follows:

| | |
|---|---|
| Ultramicrofiber (diameter: average about 20 µm) | 0.10% |
| PVA (polyvinyl alcohol) | 10.00% |
| Triton X 100 | 1.00% |
| Gelatin | 3.50% |
| H₂O | 81.40% |
| Potassium ferricyanide | 5.00% |

The hemoglobin in blood was detected for 80 seconds by the above obtained disposable non-enzymatic hemoglobin detecting electrode strip of the present invention by taking whole-blood as a sample. The test results were the same as the hemoglobin concentration detected by the cyanmet-hemoglobin method. The results show that the disposable non-enzymatic hemoglobin detecting electrode strip of the present invention can precisely detect the hemoglobin concentration in blood by taking whole-blood as a sample and does not require any pretreatment.

From the examples mentioned hereinabove, it is clear that the hemoglobin detecting electrode strip of the present invention does not require any bioactive substances and thus the production steps are simplified and shortened. Furthermore, the present invention can take whole-blood as a testing sample and precisely detect the hemoglobin concentration at a low operation voltage of below 400 mV and pH value from 5.0 to 8.0.

## Claims

1. A disposable electrode article suitable for the non-enzymatic detection of hemoglobin, said article comprising:
(1) an electric insulating substrate;
(2) a conducting film coated on one side of said insulting substrate to form an isolated and disconnected anode and an isolated and disconnected cathode;
(3) an electric insulating film coated on a part of said conducting film, wherein one portion of the conducting film anode forms an anode connector and another portion of the conducting film anode, which is uncoated by the insulating film, forms at least one reference electrode, and one portion of the conducting film cathode forms a cathode connector and another portion of the conducting film cathode, which is uncoated by the insulating film, forms at least one working electrode, and
(4) a reaction film comprising a carrier, a conductive mediator and a neutral surfactant, the surfactant being Triton X-100 having the formula 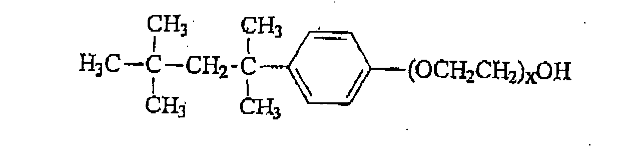
where X is from 9 to 10, the surfactant being present in a concentration of more than 1%, said reaction film being coated on a region containing at least said working electrode and said reference electrode so as to permit electrical connection of said working electrode and said reference electrode via said reaction film.

2. The electrode article of claim 1 wherein said electric insulating substrate has a flat surface.

3. The electrode article of claim 1 or 2 wherein said electric insulating substrate is thermally-resistant at temperatures of more than 40°C.

4. The electrode article of claim 3 wherein said electric insulating substrate is thermally-resistant at temperatures of 40 to 80°C.

5. The electrode article of any one of the preceding claims wherein said electric insulating substrate comprises polyvinyl chloride (PVC), fiber glass (FR-4), polyester sulphone, bakelite plate, polyethylene terephthalate (PET) plate, polycarbonate (PC) plate, glass plate or ceramics plate (CEM-1).

6. The electrode article of any one of the preceding claims, wherein said reaction film when contacted with a test sample is capable of undergoing ain electrochemical reaction.

7. The electrode article of any one of the preceding claims, wherein said reaction film is further covered with a protection film.

8. The electrode article of any one of the preceding claims, wherein said carrier comprises microcrystalline cellulose, carboxymethyl cellulose or methyl cellulose.

9. The electrode article of claim 8, wherein said carrier is microcrystalline cellulose having a size below 100 µm.

10. The electrode article of claim 8 or 9, wherein the amount of said carrier cellulose ranges from 0.01% to 0.1% by weight of the reaction film.

11. The electrode article of any one of the preceding claims, wherein said carrier optionally comprises a polymer which is polyvinyl alcohol (PVA), polyvinyl pyrrolidone (PVP), polyethylene glycol (PEG), or gelatin.

12. The electrode article of claim 11, wherein the amount of said polymer is from 0 to 15% by weight of the reaction film.

13. The electrode article of any one of the preceding claims, wherein said conductive mediator is K₃Fe(CN)₆.

14. The electrode article of any one of the preceding claims, wherein the amount of said conductive mediator is from 0.3% to 5% by weight of the reaction film.

15. The electrode article of claim 1 wherein said surfactant is present in the reaction film in an amount of 1% to 5% by weight.

16. The electrode article of claim 1, which is suitable for use in the detection of hematocrit.

17. A process for producing the electrode article as claimed in any one of the preceding claims, which process comprises:
(a) coating a conducting film on one side of an electric insulating substrate and forming an isolated and disconnected anode and an isolated and disconnected cathode;
(b) coating an electric insulating film on a part of the conducting film, wherein one portion of the conducting film anode forms an anode connector and another portion of the conducting film anode, which is uncoated by the insulating film, forms a reference electrode and one portion of the conducting film cathode forms a cathode connector and another portion of the conducting film cathode, which is uncoated by the insulating film, forms a working electrode; and
(c) forming a reaction film on a region containing at least the working electrode and the reference electrode so as to permit electrical connection of the working electrode and the reference electrode.

18. The process of claim 17 wherein the reaction film is formed by screen printing or pipetting the film on said region.

19. Apparatus suitable for the non-enzymatic detection of haemoglobin in a liquid sample, said apparatus comprising an electrode article and an amperometric sensor; wherein the electrode article comprises:
(1) an electric insulating substrate;
(2) a conducting film coated on one side of said insulting substrate to form an isolated and disconnected anode and an isolated and disconnected cathode;
(3) an electric insulating film coated on a part of said conducting film, wherein one portion of the conducting film anode forms an anode connector and another portion of the conducting film anode, which is uncoated by the insulating film, forms at least one reference electrode, and one portion of the conducting film cathode forms a cathode connector and another portion of the conducting film cathode, which is uncoated by the insulating film, forms at least one working electrode, and
(4) a reaction film comprising a carrier, a conductive mediator and a neutral surfactant, the surfactant being Triton X-100 having the formula 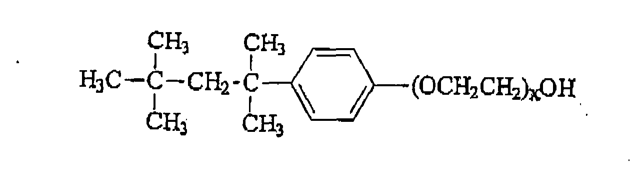
where X is from 9 to 10, the surfactant being present in a concentration of more than 1 %, said reaction film being coated on a region containing at least said working electrode and said reference electrode so as to permit electrical connection of said working electrode and said reference electrode via said reaction film; and
wherein the amperometric sensor is connected to the anode connector and the cathode connector of the electrode article, said sensor comprising a voltage output equipment, a signal receiver and a display equipment; such that the voltage output equipment is capable of providing a voltage of 100 mV to below 400 mV to the reaction film of the electrode article so as to oxidize the conductive mediator from reduction state to oxidation state after being reacted with haemoglobin of the sample; the signal receiver is capable of receiving a current, voltage or resistance change generated during a redox reaction and transmitting the change to the display equipment to display the concentration of haemoglobin in the sample.

20. The apparatus of claim 19 wherein said detecting electrode article is disposable.

21. The equipment of claim 19 or 20 adapted to measure a liquid sample which is blood.

22. A method for detecting the concentration of hemoglobin in a liquid, which comprises dropping a sample of the liquid on a disposable hemoglobin detecting electrode article of claim 1 connected to an amperometric sensor and controlling the amperometric sensor to be operated at a voltage of 100 mV to below 400 mV and a pH value of from 5.0 to 8.0.

## Patentansprüche

1. Für den nicht-enzymatischen Nachweis von Hämoglobin geeigneter Wegwerf-Elektrodengegenstand, umfassend:
(1) ein elektrisch isolierendes Substrat;
(2) einen auf eine Seite des isolierenden Substrats schichtförmig aufgetragenen leitenden Film zur Bildung einer isolierten und abgeschalteten Anode und einer isolierten und abgeschalteten Kathode;
(3) einen elektrisch isolierenden Film, der schichtförmig auf einen Teil des leitenden Films aufgetragen ist, wobei ein Bereich der leitenden Filmanode einen Anodenanschluss bildet und ein weiterer Bereich der leitenden Filmanode, der nicht mit dem isolierenden Film beschichtet ist, mindestens eine Referenzelektrode bildet und wobei ein Bereich der leitenden Filmkathode einen Kathodenanschluss bildet und ein weiterer Bereich der leitenden Filmkathode, der nicht mit dem isolierenden Film beschichtet ist, mindestens eine Arbeitselektrode bildet; und
(4) einen Reaktionsfilm, der einen Träger, einen leitenden Vermittler und ein neutrales oberflächenaktives Mittel umfasst, wobei es sich beim oberflächenaktiven Mittel um Triton X-100 der Formel 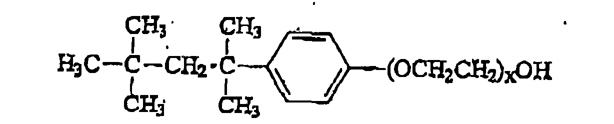 handelt, wobei X einen Wert von 9 bis 10 hat, wobei das oberflächenaktive Mittel in einer Konzentration von mehr als 1 % vorliegt, wobei der Reaktionsfilm schichtförmig auf einen Bereich aufgetragen ist, der mindestens die Arbeitselektrode und die Referenzelektrode umfasst, um einen elektrischen Anschluss der Arbeitselektrode und der Referenzelektrode über diesen Reaktionsfilm zu ermöglichen.

2. Elektrodengegenstand nach Anspruch 1, wobei das elektrisch isolierende Substrat eine flache Oberfläche aufweist.

3. Elektrodengegenstand nach Anspruch 1 oder 2, wobei das elektrisch isolierende Substrat bei Temperaturen von mehr als 40 °C wärmebeständig ist.

4. Elektrodengegenstand nach Anspruch 3, wobei das elektrisch isolierende Substrat bei Temperaturen von 40 bis 80 °C wärmebeständig ist.

5. Elektrodengegenstand nach einem der vorstehenden Ansprüche, wobei das elektrisch isolierende Substrat Polyvinylchlorid (PVC), Glasfasern (FR-4), Polyestersulfon, eine Bakelit-Dünnschicht, eine Polyethylenterephthalat (PET)-Dünnschicht, eine Polycarbonat (PC)-Dünnschicht, eine Glasdünnschicht oder eine keramische Dünnschicht (CEM-1) umfasst.

6. Elektrodengegenstand nach einem der vorstehenden Ansprüche, wobei der Reaktionsfilm bei Kontakt mit einer Testprobe einer elektrochemischen Reaktion unterliegen kann.

7. Elektrodengegenstand nach einem der vorstehenden Ansprüche, wobei der Reaktionsfilm ferner mit einem Schutzfilm bedeckt ist.

8. Elektrodengegenstand nach einem der vorstehenden Ansprüche, wobei der Träger mikrokristalline Cellulose, Carboxymethylcellulose oder Methylcellulose umfasst.

9. Elektrodengegenstand nach Anspruch 8, wobei es sich beim Träger um mikrokristalline Cellulose mit einer Größe unter 100 µm handelt.

10. Elektrodengegenstand nach Anspruch 8 oder 9, wobei die Menge der Trägercellulose im Bereich von 0,01 bis 0,1 Gew.-% des Reaktionsfilms liegt.

11. Elektrodengegenstand nach einem der vorstehenden Ansprüche, wobei der Träger gegebenenfalls ein Polymeres umfasst, bei dem es sich um Polyvinylalkohol (PVA), Polyvinylpyrrolidon (PVP), Polyethylenglykol (PEG) oder Gelatine handelt.

12. Elektrodengegenstand nach Anspruch 11, wobei die Menge des Polymeren 0 bis 15 Gew.-% des Reaktionsfilms beträgt.

13. Elektrodengegenstand nach einem der vorstehenden Ansprüche, wobei es sich beim leitenden Vermittler um K₃Fe(CN)₆ handelt.

14. Elektrodengegenstand nach einem der vorstehenden Ansprüche, wobei die Menge des leitenden Vermittlers 0,3 bis 5 Gew.-% des Reaktionsfilms beträgt.

15. Elektrodengegenstand nach Anspruch 1, wobei das oberflächenaktive Mittel im Reaktionsfilm in einer Menge von 1 bis 5 Gew,-% vorliegt.

16. Elektrodengegenstand nach Anspruch 1, der sich zur Verwendung bei der Bestimmung des Hämatokritwerts eignet.

17. Verfahren zur Herstellung des Elektrodengegenstands nach einem der vorstehenden Ansprüche, umfassend die folgenden Stufen:
(a) das schichtförmige Auftragen eines leitenden Films auf eine Seite eines elektrisch isolierenden Substrats und das Bilden einer isolierten und abgeschalteten Anode und einer isolierten und abgeschalteten Kathode;
(b) das schichtförmige Auftragen eines elektrisch isolierenden Films auf einen Teil des leitenden Films, wobei ein Bereich der leitenden Filmanode einen Anodenanschluss bildet und ein weiterer Bereich der leitenden Filmanode, der nicht mit dem isolierenden Film beschichtet ist, eine Referenzelektrode bildet und ein Bereich der leitenden Filmkathode einen Kathodenanschluss bildet und ein weiterer Bereich der leitenden Filmkathode, der nicht mit dem isolierenden Film beschichtet ist, eine Arbeitselektrode bildet; und
(c) das Bilden eines Reaktionsfilms auf einem Bereich, der mindestens die Arbeitselektrode und die Referenzelektrode enthält, um einen elektrischen Anschluss der Arbeitselektrode und der Referenzelektrode zu ermöglichen.

18. Verfahren nach Anspruch 17, wobei der Reaktionsfilm durch Siebdruck oder Pipettieren des Films auf diesem Bereich gebildet wird.

19. Vorrichtung, die zum nicht-enzymatischen Nachweis von Hämoglobin in einer flüssigen Probe geeignet ist, wobei die Vorrichtung einen Elektrodengegenstand und einen amperometrischen Sensor umfasst, wobei der Elektrodengegenstand folgendes umfasst:
(1) ein elektrisch isolierendes Substrat;
(2) einen auf eine Seite des isolierenden Substrats schichtförmig aufgetragenen leitenden Film zur Bildung einer isolierten und abgeschalteten Anode und einer isolierten und abgeschalteten Kathode;
(3) einen elektrisch isolierenden Film, der schichtförmig auf einen Teil des leitenden Films aufgetragen ist, wobei ein Bereich der leitenden Filmanode einen Anodenanschluss bildet und ein weiterer Bereich der leitenden Filmanode, der nicht mit dem isolierenden Film beschichtet ist, mindestens eine Referenzelektrode bildet und wobei ein Bereich der leitenden Filmkathode einen Kathodenanschluss bildet und ein weiterer Bereich der leitenden Filmkathode, der nicht mit dem isolierenden Film beschichtet ist, mindestens eine Arbeitselektrode bildet; und
(4) einen Reaktionsfilm, der einen Träger, einen leitenden Vermittler und ein neutrales oberflächenaktives Mittel umfasst, wobei es sich beim oberflächenaktiven Mittel um Triton X-100 der Formel 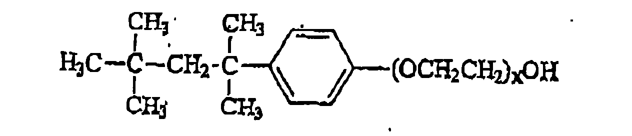
handelt, wobei X einen Wert von 9 bis 10 hat, wobei das oberflächenaktive Mittel in einer Konzentration von mehr als 1 % vorliegt, wobei der Reaktionsfilm schichtförmig auf einen Bereich aufgetragen ist, der mindestens die Arbeitselektrode und die Referenzelektrode umfasst, um einen elektrischen Anschluss der Arbeitselektrode und der Referenzelektrode über diesen Reaktionsfilm zu ermöglichen; und
wobei der amperometrische Sensor an den Anodenanschluss und den Kathodenanschluss des Elektrodengegenstands angeschlossen ist, wobei der Sensor eine Spannungsausgabeeinrichtung, einen Signalempfänger und eine Anzeigeeinrichtung umfasst, wobei die Spannungsausgabeeinrichtung zur Bereitstellung einer Spannung von 100 mV bis unter 400 mV am Reaktionsfilm des Elektrodengegenstands befähigt ist, um den leitenden Vermittler von einem Reduktionszustand in einen Oxidationszustand zu oxidieren, nachdem die Umsetzung mit Hämoglobin der Probe erfolgt ist; wobei der Signalempfänger zum Empfangen einer Strom-, Spannungs- oder Widerstandsänderung, die während einer Redoxreaktion erzeugt wird, und zur Übermittlung der Änderung zur Anzeigeeinrichtung, um die Konzentration an Hämoglobin in der Probe anzuzeigen, befähigt ist.

20. Vorrichtung nach Anspruch 19, wobei der Nachweiselektrodengegenstand für den einmaligen Gebrauch bestimmt ist.

21. Einrichtung nach Anspruch 19 oder 20, geeignet zur Messung einer flüssigen Probe, bei der es sich um Blut handelt.

22. Verfahren zum Bestimmen der Konzentration von Hämoglobin in einer Flüssigkeit, umfassend das Auftropfen einer Probe der Flüssigkeit auf einen Wegwerf-Elektrodengegenstand zur Bestimmung von Hämoglobin nach Anspruch 1, der an einen amperometrischen Sensor angeschlossen ist, und das Steuern des amperometrischen Sensors zum Betreiben bei einer Spannung von 100 mV bis unter 400 mV und einem pH-Wert von 5,0 bis 8,0.

## Revendications

1. Article d'électrode jetable approprié à la détection non enzymatique d'hémoglobine, ledit article comprenant :
(1) un substrat électriquement isolant ;
(2) un film conducteur appliqué sur une face dudit substrat isolant pour former une anode isolée et déconnectée et une cathode isolée et déconnectée ;
(3) un film électriquement isolant appliqué sur une partie dudit film conducteur, dans lequel une partie de l'anode à film conducteur forme un connecteur d'anode, et une autre partie de l'anode à film conducteur, qui n'est pas revêtue du film isolant, forme au moins une électrode de référence, et une partie de la cathode à film conducteur forme un connecteur de cathode, et une autre partie de la cathode à film conducteur, qui n'est pas revêtue du film isolant, forme au moins une électrode de travail, et
(4) un film de réaction comprenant un support, un médiateur conducteur et un agent tensioactif neutre, l'agent tensioactif étant du Triton X-100 ayant la formule 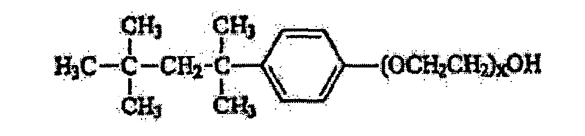
dans laquelle X vaut de 9 à 10, l'agent tensioactif étant présent à une concentration supérieure à 1 %, ledit film de réaction étant appliqué dans une région contenant au moins ladite électrode de travail et ladite électrode de référence de façon à permettre une connexion électrique de ladite électrode de travail et de ladite électrode de référence au moyen dudit film de réaction.

2. Article d'électrode selon la revendication 1, dans lequel ledit substrat électriquement isolant a une surface plane.

3. Article d'électrode selon la revendication 1 ou 2, dans lequel ledit substrat électriquement isolant est thermiquement résistant à des températures supérieures à 40 °C.

4. Article d'électrode selon la revendication 3, dans lequel ledit substrat électriquement isolant est thermiquement résistant à des températures de 40 à 80 °C.

5. Article d'électrode selon l'une quelconque des revendications précédentes, dans lequel ledit substrat électriquement isolant comprend du chlorure de polyvinyle (PVC), une fibre de verre (FR-4), de la polyestersulfone, une plaque de bakélite, une plaque de téréphtalate de polyéthylène (PET), une plaque de polycarbonate (PC), une plaque de verre ou une plaque de céramique (CEM-1).

6. Article d'électrode selon l'une quelconque des revendications précédentes, dans lequel ledit film de réaction, quand il est amené au contact d'un échantillon d'essai, est capable de subir une réaction électrochimique.

7. Article d'électrode selon l'une quelconque des revendications précédentes, dans lequel ledit film de réaction est recouvert en outre d'un film de protection.

8. Article d'électrode selon l'une quelconque des revendications précédentes, dans lequel ledit support comprend de la cellulose microcristalline, de la carboxyméthylcellulose ou de la méthylcellulose.

9. Article d'électrode selon la revendication 8, dans lequel ledit support est de la cellulose microcristalline ayant une taille inférieure à 100 µm.

10. Article d'électrode selon la revendication 8 ou 9, dans lequel la quantité de ladite cellulose de support constitue de 0,01 % à 0,1 % en poids du film de réaction.

11. Article d'électrode selon l'une quelconque des revendications précédentes, dans lequel ledit support comprend éventuellement un polymère qui est de l'alcool polyvinylique (PVA), de la polyvinylpyrrolidone (PVP), du polyéthylèneglycol (PEG) ou de la gélatine.

12. Article d'électrode selon la revendication 11, dans lequel la quantité dudit polymère est de 0 à 15 % en poids du film de réaction.

13. Article d'électrode selon l'une quelconque des revendications précédentes, dans lequel ledit médiateur conducteur est du K₃Fe(CN)₆.

14. Article d'électrode selon l'une quelconque des revendications précédentes, dans lequel la quantité dudit médiateur conducteur est de 0,3 % à 5 % en poids du film de réaction.

15. Article d'électrode selon la revendication 1, dans lequel ledit agent tensioactif est présent dans le film de réaction en une quantité de 1 % à 5 % en poids.

16. Article d'électrode selon la revendication 1, qui est approprié à une utilisation pour la détection de l'hématocrite.

17. Procédé de production de l'article d'électrode selon l'une quelconque des revendications précédentes, ledit procédé comprenant les étapes suivantes :
(a) appliquer un film conducteur sur une face d'un substrat électriquement isolant et former une anode isolée et déconnectée et une cathode isolée et déconnectée ;
(b) appliquer un film électriquement isolant sur une partie du film conducteur, dans lequel une partie de l'anode à film conducteur forme un connecteur d'anode, et une autre partie de l'anode à film conducteur, qui n'est pas revêtue du film isolant, forme une électrode de référence, et une partie de la cathode à film conducteur forme un connecteur de cathode, et une autre partie de la cathode à film conducteur, qui n'est pas revêtue du film isolant, forme une électrode de travail ; et
(c) former un film de réaction dans une région contenant au moins l'électrode de travail et l'électrode de référence de façon à permettre une connexion électrique de l'électrode de travail et de l'électrode de référence.

18. Procédé selon la revendication 17, dans lequel le film de réaction est formé par application du film sur ladite région par sérigraphie ou à la pipette.

19. Appareil approprié pour la détection non enzymatique d'hémoglobine dans un échantillon liquide, ledit appareil comprenant un article d'électrode et un capteur ampérométrique, dans lequel l'article d'électrode comprend :
(1) un substrat électriquement isolant ;
(2) un film conducteur appliqué sur une face dudit substrat isolant pour former une anode isolée et déconnectée et une cathode isolée et déconnectée ;
(3) un film électriquement isolant appliqué sur une partie dudit film conducteur, dans lequel une partie de l'anode à film conducteur forme un connecteur d'anode, et une autre partie de l'anode à film conducteur, qui n'est pas revêtue du film isolant, forme au moins une électrode de référence, et une partie de la cathode à film conducteur forme un connecteur de cathode, et une autre partie de la cathode à film conducteur, qui n'est pas revêtue du film isolant, forme au moins une électrode de travail, et
(4) un film de réaction comprenant un support, un médiateur conducteur et un agent tensioactif neutre, l'agent tensioactif étant du Triton X-100 ayant la formule 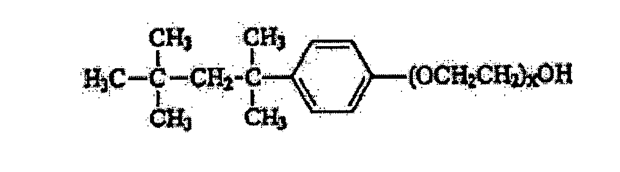
où X vaut de 9 à 10, l'agent tensioactif étant présent à une concentration supérieure à 1 %, ledit film de réaction étant appliqué sur une région contenant au moins ladite électrode de travail et ladite électrode de référence de façon à permettre une connexion électrique de ladite électrode de travail et de ladite électrode de référence au moyen dudit film de réaction ; et
dans lequel le capteur ampérométrique est connecté au connecteur d'anode et au connecteur de cathode de l'article d'électrode, ledit capteur comprenant un équipement de tension de sortie, un récepteur de signaux et un équipement d'affichage, de façon à ce que l'équipement de tension de sortie soit capable de fournir une tension de 100 mV à moins de 400 mV au film de réaction de l'Article d'électrode de façon à oxyder le médiateur conducteur d'un état de réduction à un état d'oxydation après avoir été mis en réaction avec de l'hémoglobine de l'échantillon ; le récepteur de signaux est capable de recevoir un changement de courant, de tension ou de résistance généré pendant une réaction d'oxydoréduction et de transmettre le changement à l'équipement d'affichage pour afficher la concentration d'hémoglobine dans l'échantillon.

20. Appareil selon la revendication 19, dans lequel ledit article d'électrode de détection est jetable.

21. Équipement selon la revendication 19 ou 20, adapté pour mesurer un échantillon liquide qui est du sang.

22. Procédé de détection de la concentration d'hémoglobine dans un liquide, qui comprend faire couler goutte à goutte un échantillon du liquide sur un article d'électrode jetable à détection d'hémoglobine selon la revendication 1, connecté à un capteur ampérométrique, et à contrôler le capteur ampérométrique pour qu'il soit actionné à une tension de 100 mV à moins de 400 mV et à une valeur de pH de 5,0 à 8,0.
